# EUROPEAN PATENT SPECIFICATION

(11) **EP 1 103 253 B1**
(45) Date of publication and mention of the grant of the patent: **08.03.2006**
(21) Application number: 00403170.4
(22) Date of filing: 14.11.2000
(51) Int. Cl.: A61K 9/28

(54) **Aqueous film coating agent and oral solid preparation**
Wässriges filmbildendes Überzugsmittel und oral anzuwendende feste Arzneizubereitung
Agent filmogène aqueux de revêtement et préparation solide à utilisation orale

(30) Priority: 19.11.1999 JP 32955299
(43) Date of publication of application: 30.05.2001
(73) Proprietor: Shin-Etsu Chemical Co., Ltd., Chiyoda-ku, Tokyo (JP)
(72) Inventor: Kokubo, Hiroyasu, Chiyoda-ku, Tokyo (JP); Tanno, Fumie, Kubiki-gun, Niigata-ken (JP); Nishiyama, Yuichi, Kubiki-gun, Niigata-ken (JP)
(74) Representative: Rémont, Claude

(56) References cited:
- EP-A- 0 753 262
- EP-A- 0 839 527
- WO-A-95/06462
- DATABASE WPI Section Ch, Week 198510 Derwent Publications Ltd., London, GB; Class A96, AN 1985-058370 XP002168664 & JP 60 013719 A (SHINETSU CHEM IND CO LTD), 24 January 1985 (1985-01-24)

## Description

### BACKGROUND OF THE INVENTION

### 1. Field of the Invention

The present invention relates to an aqueous film coating agent which masks uncomfortable tastes such as bitterness of a tablet and excels in an dissolution property of a drug, and an oral solid preparation coated with such a film coating agent.

### 2. Description of the related art

An aqueous film coating is widely used in the field of an oral solid preparation for purposes of masking of an uncomfortable taste of a drug, a shade for keeping a stability of a drug, a color coating for discrimination of oral solid preparations more clearly and prevention from attrition of an oral solid preparation in transport. Among them, a coating method using an aqueous solution of hydroxypropylmethylcellulose (hereinafter, also referred to as "HPMC") is most widely used.

Initially (in 1950s) when a film coating was developed, the degree of polymerization (viscosity) of a substrate, HPMC was high so that an organic solvent was used. In 1970s, the substrate having a lower viscosity was developed, and simultaneously, a perforation type coating apparatus came on, leading gradually to emergence of an aqueous film coating.

Such an aqueous film coating with HPMC thus developed has been established as an excellent coating method since such a film is excellent in flexibility and strength. Moreover, the aqueous film coating has a characteristic that a drug therein is quickly dissolved and eluted in water or various buffer solutions.

However, since HPMC is quickly dissolved in water, an oral solid preparation being film-coated with HPMC is rapidly dissolved in a mouth. Thus, masking of bitterness is insufficient.

For obtaining stronger masking of bitterness or stronger moisture-proof coating, a pH-dependent acrylic or vinyl-based polymer is sometimes used instead of HPMC. However, these polymers are of an acid-soluble type and have a problem that the coated film thereof passes through stomach without being dissolved in the stomach of a patient suffering from anacidity (achlorhydria).

On the other hand, methylcellulose (hereinafter, also referred to as "MC") is a polymer appeared first as a water-soluble cellulose ether, and the gelling temperature thereof is usually not more than 37°C at which examination of a preparation is conducted. This polymer causes heat gelling and can not be dissolved under an environment at 37°C. Hence, methylcellulose is not used in a film coating, and exclusively used for a binding agent and a suspension stabilizer.

As a special example in which MC is used in a film coating, an aqueous film coating on granules has been introduced, utilizing low gelling temperature and low adhesion of MC (Chem. Pharm. Bull., 46(11) 1803-1806 (1998)).

Japanese Patent Publication No. 60013715 has proposed a film coating of MC which does not decompose in an oral cavity, taking an advantage of the gelling property thereof.

Further, Japanese Patent Publication No.60084215 has proposed a method for improving slime-like feeling in an oral cavity, when a tablet coated with a HPMC film is dosed. This method is based on an addition of MC to HPMC.

Though such an application of MC to a film coating is possible, there is a fear of delay in dissolution of a drug since heat gelling occurs at 37°C as described above.

Recently, bioavailability of a drug has become a matter of significance, and an dissolution test of a solid preparation such as a tablet has been retraced from the standpoint of evaluations for effective use and equivalence of drugs. It is believed that a preparation with rapid dissolution is endowed with effectiveness or equivalence. Accordingly, there is required a preparation which can have rapid dissolution under a more gentle condition (a paddle method: 50 rpm).

For solving these problems, Japanese Patent Publication No. 11060472 has proposed a film coating composition which improves sticky feeling and slime-like feeling in an oral cavity, intending smooth swallowing. It is based on the addition of saccharide into MC. However, this film coating composition is for an organic solvent-based coating composed of a water-alcohol system. Thus, it is not preferable from the environmental point of view since a solvent used is discharged into air, and requires a solvent - recovering apparatus for preventing this. Further, MC to be used has a viscosity of 25 mm²/s or less in an aqueous solution of 2% by weight thereof.

### SUMMARY OF THE INVENTION

Thus, an object of the present invention is to provide an aqueous film coating agent which uses no organic solvent, excels in masking of bitterness and improves a eluting property as compared with a conventional film coating agent, and an oral solid preparation coated with such a film coating agent.

The present inventors have intensively investigated these problems for a solution and found that an aqueous film coating agent comprising methylcellulose having a viscosity of 2.0 to 8.0 mm²/s in an aqueous solution of 2% by weight of the methylcellulose at 20°C, and saccharide or sugar alcohol, can excel in masking of bitterness and improve an dissolution property of a drug without using of organic solvent, leading to completion of the present invention.

According to the aqueous film coating agent of the present invention, excellent masking of bitterness of a drug is obtained and slime-like feeling and sticky feeling are suppressed when the oral solid preparation is dosed. Further, rapid dissolution of a drug can be maintained even when coated therewith.

### DETAILED DESCRIPTION OF THE PREFERRED EMBODIMENTS

The present invention is illustrated in detail below through preferable embodiments of the present invention.

MC having a low viscosity used in the present invention is produced by reducing the polymerization degree of the raw material, commercially available MC or MC synthesized by a known method, with an aqueous hydrochloric acid (Japanese Patent Publication (JP-A) No.= 62025101 European Patent Publication (EP-A) No. 210917) or hydrochloric acid gas (Japanese Patent Publication (JP-B) No. 48-41037, US Patent No. 3391135).

Commercially available MC usually has a viscosity of about 15 mm²/s in an aqueous solution of 2% by weight thereof at 20°C by the viscosity measurement defined in the Japanese Pharmacopoeia.

The viscosity of MC used in the present invention is preferably lower than that of commercially available MC so that an aqueous coating solution can be sprayed as a high concentration solution of MC. It is preferably in the range of 2.0 to 8.0 mm²/s, more preferably 2.0 to 5.0 mm²/s. When it is less than 2.0 mm²/s, strength of a film may not be kept enough due to extreme reduction in the polymerization degree of MC. On the other hand, when it is more than 8.0 mm²/s, the concentration of the aqueous coating solution may have to be lowered to the extent of unpracticality.

In the present invention, a water-soluble component is added for improving the dissolution property. The water-soluble component may advantageously be one which exerts no influence on heat gelling temperature of MC, and saccharide or sugar alcohol in the present invention does not exert influence on heat gelling temperature of MC.

The above-mentioned saccharide or sugar alcohol can be added alone or in admixture to the above-mentioned MC.

The saccharide in the present invention includes monosaccharide, disaccharide and polysaccharide. Specific examples thereof include saccharose, lactose, glucose, fructose, maltose, trehalose, maltodextrin and polydextrose. The sugar alcohol in the present invention includes erythritol, sorbitol, xylytol, mannitol and maltitol.

The amount of the saccharide or sugar alcohol to be added is preferably in 10 to 200% by weight, more preferably 30 to 100% by weight based on MC, though it varies depending on the solubility of saccharide or sugar alcohol. When the amount of saccharide or sugar alcohol is low, rapid dissolution of a drug may not be obtained easily. When the amount thereof is high, film strength may decrease, leading to a trouble in coating. This is particularly remarkable in the case of a film coating agent having a pigment.

The aqueous film coating agent obtained by adding saccharide or sugar alcohol into MC as described above can be dissolved in water at a concentration of 5 to 15% by weight and coated on a-solid preparation. For this coating, there can be used an apparatus generally used for production of pharmaceuticals such as a pan coating apparatus, perforation type drum coating apparatus or a fluidized bed coating apparatus.

The aqueous film coating of the present invention may comprise a plasticizer or a pigment. The plasticizer which can be used in the coating of the present invention includes polyethylene glycol, glycerin, propylene glycol, triacetin, triethyl citrate. It is preferably polyethylene glycol or glycerin in a liquid form which has an excellent compatibility with MC.

The amount of the above-mentioned plasticizer to be added is generally increased in the composition containing a large amount of pigment. Usually, the amount of the plasticizer is preferably from 3 to 50% by weight based on MC.

Further, the above-mentioned pigment include titanium oxide or aluminum lake . The amount thereof to be added varies depending on shading or coloring which is an object of the addition, and preferably in the range of 3 to 30% by weight based on MC.

The aqueous film coating agent of the present invention may further comprise an additive generally used on coating such as a coloring agent; a stickiness preventing agent such as talc, polyethylene glycol in a solid form, aerosil (silicon dioxide) or titanium dioxide; a silicone-based de-foaming agent such as KM-72 (manufactured by Shin-Etsu Chemical Co., Ltd.); a polyoxyethylenepolyoxypropylene glycol-based de-foaming agent such as Pullonic F68 (manufactured by Asahi Denka Kogyo K.K.); a de-foaming agent such as sorbitan sesquioleate; or a perfume such as spearmint or menthol.

The amount coated on the solid preparation differs significantly depending on a size and form of a tablet or a granule, and a kind of a drug to be masked. In the case of a tablet, the amount may be same or slightly higher than that for a general film coating in order to obtain the advantages of the present invention.

The aqueous film coating agent of the present invention can also be used for undercoating of a sugar-coated tablet or an enteric tablet in the same manner as a conventional HPMC film coating.

The following examples and comparative examples illustrate the present invention further in detail.

### <Example 1>

### Preparation of Tablets:

50 parts by weight of anhydrous caffeine (manufactured by Shiratori Seiyaku K.K.) obtained through a No. 30 (500 µm) sieve, 139 parts by weight of granulated lactose (DLC 11: manufactured by DMV Co., Ltd.) and 10 parts by weight of low-substituted hydroxypropylcellulose (LH-11: manufactured by Shin-Etsu Chemical Co., Ltd.) were mixed in a V-shape mixer, and further mixed after the addition of 1 part by weight of magnesium stearate (SM-1000: Sakai Chemical Industry Co., Ltd.) thereto. Then, the resulted mixture was tableted by a high speed tableting machine (VIRG: manufactured by Kikusui Seisakusho K.K.) using punches having a diameter of 8 mm and an ordinary concave so that the weight per table was 200 mg.

The disintegrating time of the resulted tablet was measured by using purified water as a test solution and using no disk according to a disintegrating test method defined in the Japanese Pharmacopoeia of Japan. It was 13 minutes. Coating:

6 parts by weight of methylcellulose (Metolose SM-4: manufactured by Shin-Etsu Chemical Industry Co., Ltd.; viscosity of an aqueous solution of 2% by weight thereof at 20°C: 4.2 mm²/s), 2 parts by weight of erythritol (manufactured by Nikken Chemicals Co., Ltd.) and 2 parts by weight of glycerin (manufactured by Wako Pure Chemical Industries Ltd.) were dissolved in 90 parts by weight of purified water to yield a coating solution.

1.2 kg of the caffeine tablets prepared in the above were placed in a small size perforation type coating apparatus (manufactured by Shin-Etsu Chemical Industry Co., Ltd.). A coating solution was sprayed thereon at a rate of 6g per minute at a gas drying air temperature of 80°C so that coated tablets having 6 mg of coating per tablet were obtained.

### Dissolution Test:

The resulted coated tablet was tested by a paddle method at 50 rpm using purified water as a test solution according to a dissolution test method defined in the Japanese Pharmacopoeia, to measure the dissolution speed of caffeine. The result is shown in Table 1.

**Table 1**

| | dissolution rate (%) | | | | | |
|---|---|---|---|---|---|---|
| | 5 minutes | 10 minutes | 15 minutes | 20 minutes | 30 minutes | 45 minutes |
| Example 1 | 20 | 52 | 69 | 81 | 99 | 100 |
| Example 2 | 41 | 65 | 78 | 87 | 100 | 100 |
| Example 3 | 33 | 49 | 67 | 80 | 98 | 100 |
| Example 4 | 38 | 61 | 74 | 88 | 100 | 100 |
| Comparative Example 1 | 27 | 68 | 85 | 92 | 100 | 100 |
| Comparative Example 2 | 42 | 55 | 76 | 90 | 98 | 100 |
| Comparative Example 3 | 0 | 0 | 0 | 2 | 25 | 55 |
| tablet not coated | 48 | 70 | 84 | 93 | 100 | 100 |

### Sensuality Test:

The resulted coating tablets were dosed to 12 volunteers in the rate of one tablet each person. When the tablet was moved slowly in an oral cavity, the time until bitterness was felt was measured. The average value thereof was calculated. The presence or absence of slime-like feeling were also judged for the dose and the results are shown in Table 2.

**Table 2**

| | Time until bitterness was felt | feeling on tongue (slime-like feeling) |
|---|---|---|
| Example 1 | 30 seconds | None |
| Example 2 | 26 seconds | None |
| Example 3 | 35 seconds | None |
| Example 4 | 29 seconds | None |
| Comparative Example 1 | 12 seconds | Recognized |
| Comparative Example 2 | 40 seconds | None |
| Comparative Example 3 | 1 minute or more | None |

### <Example 2>

### Preparation of Tablets:

The same tablets as used in Example 1 were used. Coating:

6 parts by weight of methylcellulose (Metolose SM-4: manufactured by Shin-Etsu chemical Industry Co., Ltd.; viscosity of an aqueous solution of 2% by weight thereof at 20°C: 4.2 mm²/s), 6 parts by weight of xylytol (manufactured by Tokyo Kasei K.K.) and 3 parts by weight of Macrogol 400 (manufactured by Nippon oil and Fat Co., Ltd.) were dissolved in 85 parts by weight of purified water to yield a coating solution. Coated tablets having 6mg of the coating per tablet were obtained under the same coating condition as in Example 1.

### Dissolution Test:

Results of the dissolution test conducted in the same manner as in Example 1 are shown in Table 1.

### Sensuality Test:

Results of the sensuality test conducted in the same manner as in Example 1 are shown in Table 2.

### <Example 3>

### Preparation of Tablets:

The same tablets as used in Example 1 were used. Coating:

5 parts by weight of methylcellulose (manufactured by Shin-Etsu Chemical Industry Co., Ltd.; viscosity of an aqueous solution of 2% by weight thereof at 20°C: 6.2 mm²/s), 3 parts by weight of granulated sugar (manufactured by Nissin Sugar Manufacturing Co., Ltd.) and 2 parts by weight of glycerin (manufactured by Wako Pure Chemical Industries Ltd.) were dissolved in 90 parts by weight of purified water to yield a coating solution. Coating tablets having 6mg of coating per tablet were obtained under the same coating condition as in Example 1.

### Dissolution Test:

Results of the dissolution test conducted in the same manner as in Example 1 are shown in Table 1.

### Sensuality Test:

Results of the sensuality test conducted in the same manner as in Example 1 are shown in Table 2.

### <Example 4>

### Preparation of Tablets:

The same tablets as used in Example 1 were used. Coating:

6 parts by weight of methylcellulose (manufactured by Shin-Etsu Chemical Industry Co., Ltd.; viscosity of an aqueous solution of 2% by weight thereof at 20°C: 6.2 mm²/s), 6 parts by weight of lactose (Pharmatose 100M: manufactured by DMV) and 3 parts by weight of glycerin (manufactured by Wako Pure Chemical Industries Ltd.) were dissolved in 85 parts by weight of purified water to yield a coating solution. A coating tablets having 6 mg of coating per tablet were obtained under the same coating condition as in Example 1.

### Dissolution Test:

Results of the dissolution test conducted in the same manner as in Example 1 are shown in Table 1.

### Sensuality Test:

Results of the sensuality test conducted in the same manner as in Example 1 are shown in Table 2.

### <Comparative Example 1>

### Preparation of Tablets:

The same tablets as used in Example 1 were used. Coating:

7 parts by weight of hydroxypropylmethylcellulose (TC-5R: manufactured by Shin-Etsu Chemical Industry Co., Ltd.; viscosity of an aqueous solution of 2% by weight thereof at 20°C: 5.9 mm²/s) and 1 part by weight of Macrogol 6000 (manufactured by Nippon Oil and Fat Co., Ltd.) were dissolved in 92 parts by weight of purified water to yield a coating solution. Coating tablets having 6.mg of coating per tablet were obtained under the same coating condition as in Example 1.

### Dissolution Test:

Results of the dissolution test conducted in the same manner as in Example 1 are shown in Table 1.

### Sensuality Test:

Results of the sensuality test conducted in the same manner as in Example 1 are shown in Table 2.

### <Comparative Example 2>

### Preparation of Tablets:

The same tablets as used in Example 1 were used. Coating:

3 parts by weight of methylcellulose (Metolose SM-15: manufactured by Shin-Etsu Chemical Industry Co., Ltd.; viscosity of an aqueous solution of 2% by weight at 20°C: 15.6 mm²/s), 1 part by weight of erythritol (manufactured by Nikken Chemicals Co., Ltd.) and 1 part by weight of glycerin (manufactured by Wako Pure Chemical Industries Ltd.) were dissolved in 95 parts by weight of purified water to yield a coating solution. A coating tablets having 6mg of coating per tablet were obtained under the same coating condition as in Example 1.

### Dissolution Test:

Results of the dissolution test conducted in the same manner as in Example 1 are shown in Table 1.

### Sensuality Test:

Results of the sensuality test conducted in the same manner as in Example 1 are shown in Table 2.

### <Comparative Example 3>

### Preparation of Tablets:

The same tablets as used in Example 1 were used. coating:

8 parts by weight of methylcellulose (Metolose SM-4: manufactured by Shin-Etsu Chemical Industry Co., Ltd.; viscosity of an aqueous solution of 2% by weight thereof at 20°C: 4.2 mm²/s) and 2 parts by weight of glycerin (manufactured by Wako Pure Chemical Industries Ltd.) were dissolved in 90 parts by weight of purified water to yield a coating solution. A coated tablets having 6 mg of coating per tablet were obtained under the same coating condition as in Example 1.

### Dissolution Test:

Results of the dissolution test conducted in the same manner as in Example 1 are shown in Table 1.

### Sensuality Test:

Results of the sensuality test conducted in the same manner as in Example 1 are shown in Table 2.

### <Example 5>

### Preparation of Tablets:

10 parts by weight of ibuprofen (manufactured by Yonezawahama Riyakuhin Kogyo K.K.) obtained through a No. 30 (500 µm) sieve, 79 parts by weight of granulated lactose (DLC 11: manufactured by DMV Co., Ltd.) and 20 parts by weight of low-substituted hydroxypropylcellulose (LH-11: manufactured by Shin-Etsu Chemical Co., Ltd.) were mixed in a V-shape mixer and further mixed after 1 part by weight of magnesium stearate (SM-1000, Sakai Chemical Industry Co., Ltd.). Then, the resulted mixture was tableted by a high speed tableting machine (VIRG: manufactured by Kikusui Seisakusho K.K.) using punches having a diameter of 8 mm and ordinary concave. The of the weight each tablet was 200 mg.

The disintegrating time for the resulted tablet was measured by using purified water as a test solution and using no disk according to a disintegrating test method defined in The Japanese Pharmacopoeia. It was 7 minutes.

### Coating:

A coated tablets having 6 mg of coating per tablet were obtained under the same coating condition as in Example 1 using the same composition as in Example 1.

### Dissolution Test:

Results of the dissolution test conducted in the same manner as in Example 1 are shown in Table 3.

**Table 3**

| | dissolution rate (%) | | | | | |
|---|---|---|---|---|---|---|
| | 5 minutes | 10 minutes | 15 minutes | 20 minutes | 30 minutes | 45 minutes |
| Example 5 | 38 | 78 | 87 | 97 | 100 | 100 |
| Example 6 | 33 | 64 | 85 | 96 | 100 | 100 |
| tablet not coated | 73 | 99 | 100 | 100 | 100 | 100 |

### Sensuality Test:

Results of the sensuality test conducted in the same manner as in Example 1 are shown in Table 4.

**Table 4**

| | Time until bitterness was felt | Feeling on togue (slime-like feeling) |
|---|---|---|
| Example 5 | 27 seconds | none |
| Example 6 | 28 seconds | none |

### Example 6

### Preparation of Tablets:

The same tablet as used in Example 5 was used.

### Coating:

6 parts by weight of methylcellulose (Metolose SM-4: manufactured by Shin-Etsu Chemical Industry Co., Ltd.; viscosity of an aqueous solution of 2% by weight thereof at 20°C: 4.2 mm²/s) and 2 parts by weight of erythritol (manufactured by Nikken Chemicals Co., Ltd.) were dissolved in 92 parts by weight of purified water to yield a coating solution. A coated tablets having 6 mg of coating per tablet were obtained under the same coating condition as in Example 1.

### Dissolution Test:

Results of the dissolution test conducted in the same manner as in Example 1 are shown in Table 3.

### Sensuality Test:

Results of the sensuality test conducted in the same manner as in Example 1 are shown in Table 4.

As described above, the conventional film coating as in Comparative Example 1 had insufficient masking of bitterness and caused slime-like feeling in an oral cavity. When MC having high viscosity as in Comparative Example 2 was used, the coated tablets excelled in properties. However, concentration of a coating solution was low so that twice as much coating time was needed for obtaining a tablet having the same coating amount as the present invention. In the formulation of Comparative Example 3 having only MC without saccharide or sugar alcohol, dissolution of a drug was delayed remarkably. -

## Claims

1. An aqueous film coating agent comprising methylcellulose having a viscosity of 2.0 to 8.0 mm²/s in an aqueous solution of 2% by weight thereof at 20°C, and saccharide or sugar alcohol.

2. An oral solid preparation coated with the aqueous film coating agent according to Claim 1.

## Patentansprüche

1. Wäßriges Filmbeschichtungsreagenz, aufweisend Methylcellulose mit einer Viskosität von 2,0 bis 8,0 mm²/s in einer 2 Gew.-% enthaltenen wäßrigen Lösung bei 20°C und Saccharide oder Zuckeralkohol.

2. Eine feste orale Zubereitung beschichtet mit einem wäßrigen Filmbeschichtungsreagenz gemäß Anspruch 1.

## Revendications

1. Agent filmogène aqueux de revêtement comprenant de la méthylcellulose ayant une viscosité de 2,0 à 8,0 mm²/s dans une solution aqueuse de 2 % en poids de celle-ci à 20 °C, et un saccharide ou un polyol.

2. Préparation solide orale enrobée avec l'agent filmogène aqueux de revêtement selon la revendication 1.
